# EUROPEAN PATENT SPECIFICATION

(11) **EP 2 076 588 B1**
(45) Date of publication and mention of the grant of the patent: **21.08.2013**
(21) Application number: 07820134.0
(22) Date of filing: 11.09.2007
(51) Int. Cl.: C12N 5/0775

(54) **EXPANSION METHOD FOR ADULT STEM CELLS FROM BLOOD, PARTICULARLY PERIPHERAL BLOOD, AND RELATIVE APPLICATION IN MEDICAL FIELD**
EXPANSIONSVERFAHREN FÜR ADULTE STAMMZELLEN AUS BLUT, BESONDERS PERIPHEREM BLUT, UND DAMIT VERBUNDENE ANWENDUNG AUF MEDIZINISCHEM GEBIET
PROCÉDÉ D'EXPANSION DE CELLULES SOUCHES ADULTES À PARTIR DE SANG, NOTAMMENT DE SANG PÉRIPHÉRIQUE, ET APPLICATION CONNEXE DANS LE DOMAINE MÉDICAL

(30) Priority: 20.09.2006 IT RM20060498
(43) Date of publication of application: 08.07.2009
(73) Proprietor: THANKSTEM S.R.L., 33100 Udine (UD) (IT)
(72) Inventor: GAMBACURTA, Alessandra, I-00132 Roma (IT); POLETTINI, Marco, I-01015 Sutri (IT)
(74) Representative: Petraz, Gilberto Luigi
(86) International application number: PCT/EP2007/059531
(87) International publication number: WO 2008/034740

(56) References cited:
- WO-A-03/083092
- WO-A-2004/043990
- ZHAO YONG ET AL: "A human peripheral blood monocyte-derived subset acts as pluripotent stem cells." PROCEEDINGS OF THE NATIONAL ACADEMY OF SCIENCES OF THE UNITED STATES OF AMERICA 4 MAR 2003, vol. 100, no. 5, 4 March 2003 (2003-03-04), pages 2426-2431, XP002410596 ISSN: 0027-8424 cited in the application
- SMITH R K W ET AL: "Isolation and implantation of autologous equine mesenchymal stem cells from bone marrow into the superficial digital flexor tendon as a potential novel treatment." EQUINE VETERINARY JOURNAL JAN 2003, vol. 35, no. 1, January 2003 (2003-01), pages 99-102, XP008066230 ISSN: 0425-1644
- SMITH AUSTIN: "A glossary for stem-cell biology", NATURE (LONDON),, vol. 441, no. 7097, 1 June 2006 (2006-06-01), page 1060, XP002616488,

## Description

The present invention concerns an expansion method for stem cells from blood, particularly but not only peripheral blood, of adult mammals, and the relative application in the medical field, in particular in the veterinary field, for the treatment of lesions, chronic and/or acute inflammatory pathologies, neurological and neurodegenerative pathologies.

Here, and hereafter in the description, and as is known in literature, by the word "expansion" we mean the process of increasing the number of cells either by cell division or, as in the specific case described and claimed, by "de-differentiation", that is, the process by which the cells present in the blood are transformed into stem cells following suitable in-vitro treatment, as will be seen hereafter.

In recent years the use of stem cells in therapy has had great approval due to the successes obtained in treating various pathologies which were previously thought incurable. However, processes for obtaining stem cells known until now have been laborious and expensive.

Pluripotent stem cells (PSC) are a source available not only for research but also for the creation of drugs and for transplants (Wagers A. J. et al., 2002; Griffith L. G. et al., 2002).

There are two ample categories of stem cells, embryonic and adult: the first derive from embryos, and more exactly from 8-day blastocysts, while on the contrary adult stem cells can be obtained mainly from bone marrow, adipose or muscular tissue and from peripheral blood.

The definition of stem cell is constantly evolving and, at the moment, there is no general consensus or standard method to isolate or identify them. For all these cells, embryonic (ES) and adult, both hematopoietic (HSC) and mesenchymal (MSC) (Kuwana M. et al., 2003), different genetic markers have been identified, of which some are common to many cell types (Condomines M. et al., 2006; Kang W. J. et al., 2006; Zhao Y. et al., 2003; Rabinovitch M. et al., 1976).

In particular, Zhao Y. et al, both in the Article *"A human peripheral blood monocyte-derived subset acts as pluripotent stem cells"* and in WO 2004/043990, discloses a method for preparing monocyte-derived stem cells which includes the steps of isolating a peripheral-blood monocyte, contacting it with a mitogenic component and subsequently culturing the peripheral-blood monocyte under conditions suitable for the propagation of the cells.

This method, which requires firstly a step of isolating the monocyte and secondly an expansion step in a culturing media, involves very long times to obtain a significant number of stem cells, in the order of 15-20 days, and is not able to obtain stem cells of the totipotent type, that is, non-specialized, suitable to be directly inoculated in the patient in a very short time from the first drawing.

Numerous scientific works describe stem cells' ability to regenerate different types of lesions, regenerating tissues that are mechanically damaged or damaged by various pathologies, eliminating at the root the causes that generated the pathology and not simply acting on the effects thereof.

At the moment, research is more oriented towards the use of stem cells isolated from embryonic tissue, fetuses or the umbilical cord, but this is raising various legal and ethical questions. Above all, as of today the use of these cells brings various contraindications such as: risks of infection, of rejection if transplanted and, in horses, the onset of teratomas.

To obviate these problems it has therefore been thought to use in the "in vivo" therapy autologous stem cells isolated preferably from bone marrow, adipose tissue or peripheral blood. These methods, used starting from adult stem cells, provide a step of differentiation "in-vitro" (or "ex vivo") of the stem cells in the cell line desired by means of specific differentiation induction factors, and a subsequent step of "in vivo" transplantation of the differentiated cell line obtained. In these methods the limit is due to fact that observable rejection phenomena occur because the differentiated cells re-introduced into the patient are not recognized as self-cells, because during the differentiation step induced in-vitro they lose the self-recognition factors.

In man, taking stem cells from peripheral blood entails purifying them through a process called "aphaeresis" or "leucophaeresis". In practice, the cells are extracted from the blood, collected, and then inoculated into patients immediately after chemo- or radio therapy. In aphaeresis, which lasts from 6 to 8 hours, the blood is taken from the vein of an arm or a vein in the neck or the chest, and made to pass through a machine which removes the stem cells. The blood, thus purified, returns to the patient, while the cells collected are preserved by means of refrigeration in liquid nitrogen (Condomines M. et al., 2006; Kang W.J. et al., 2006). This technique is not only painful, but also extremely stressful for the patient. Furthermore, it is impracticable for animals of either small or large size; above all, the technique does not provide a real discrimination and/or purification of the stem cells circulating.

At present, in veterinary science, stem cells are used successfully mainly in the reconstruction of tendons and ligaments with lesions. The main techniques for purification are:
- use of growth factors or platelet derivatives (TGF-B, VEGF), but the economic costs of extracting these are prohibitive (Hou M. et al., 2006);
- isolation of stem cells taken from bone marrow. This technique allows to purify and then use for therapy only 15% of the cells contained in the material extracted;
- isolation of stem cells taken from adipose tissue. This technique, which requires the prior surgical removal of considerable quantities of tissue from the donor animal, does not allow intravenous administration;
- IGF-1 (insulin-like growth factor 1) known as Tendotrophin (Fiedler J. et al., 2006);
- UBM (urinary bladder matrix): this is a derivative from the pig containing cytokines (but not nucleate cells), which induce the cicatrization of the wound but not the regeneration of the zone with lesions (Zhang Y.S. et al., 2005).

In the light of all the above, it is obvious that methods are needed for the expansion and purification of adult stem cells from easily accessible sources which must also allow to obtain stem cells suitable for use as medication in the medical-veterinary field which, once administered in the mammal, do not give rise to phenomena of rejection and which are easy to preserve.

There is also an obvious need to obtain stem cells of the pluripotent and totipotent type, that is, non-specialized, which can be inoculated directly into the patient, and with much shorter production times than those provided at present.

The authors of the present invention have now perfected a method for the expansion and purification in-vitro of stem cells from peripheral blood such as to allow to obtain stem cells which do not give collateral effects such as phenomena of rejection, infection, teratomas, once administered in the adult mammal, able to be differentiated "in vivo" and to behave as pluripotent stem cells.

The authors have seen that the cells thus expanded, once injected locally or intravenously, acquire "in vivo" (and not "in-vitro", as in known methods in the state of the art by means of suitable growth factors and/or chemical stimuli (Gulati R. et al., 2003; Katz R. L. et al., 2002; Okazaki T. et al., 2005)) all the morphological and chemical characteristics of macrophagic, lymphocytic, epithelial, endothelial, neuronal and hepatocyte cells, according to the needs and pathologies of the animals treated. The method is even less invasive than other methods used until now to collect stem cells, painless (if compared with aphaeresis), economical and technically the most suitable to be used in all animal species (small and large).

Finally, the possibility of obtaining these cells easily, and then being able to preserve them for a long time refrigerated in liquid nitrogen, makes the cells obtained with the method according to the invention suitable for autologous transplants, for the treatment of many human and veterinary pathologies (lesions of various type, metabolic illnesses, acute and chronic neurological and inflammatory pathologies).

The present invention therefore specifically concerns a method for the expansion of adult stem cells from blood, particularly but not only peripheral blood, comprising the following steps.

A first step comprises two substeps:
a) a first substep of expansion of the stem cells of blood, particularly but not only peripheral blood, immediately after they have been taken, by means of in-vitro treatment with MCSF (Macrophage Colony Stimulating Factor) in a concentration comprised between 8-15 nM, preferably 10 nM; the expansion step may have a variable duration according to the conditions in which the in-vitro treatment is carried out; the authors have verified experimentally that a duration of the in-vitro treatment with MCSF comprised between 24 and 96 hours, advantageously between 48 and 72 hours, has led to a stabilization of the expansion, with identification of the simultaneous presence of the markers CD 90, CD34 and mixed CD 90/CD 34. This condition was considered the optimum one.
   By "immediately" we mean the shortest possible time between the blood cells being taken and the start of the in-vitro treatment, in any case not more than 10 minutes, advantageously not more than 5 minutes.
b) after the first substep, a second substep of purification, preferably by means of fractioning on a Ficoll gradient.
   The purification step is fundamentally intended to destroy the red corpuscles and to cultivate the macrophages and monocytes on a plate.

Therefore, contrary to the state of the art the present invention provides firstly a step of expansion of the cells, by contacting them with MCSF and a possible anti-coagulant product, for example in a suitable test tube; this expansion step is performed immediately after the cells of blood have been taken from the patient, without isolating specific parts of them and without using any culturing media.

After the step of purification, another step is provided of:
c) further expansion of the stem cells of blood, particularly but not only peripheral blood, purified in step b) by means of in-vitro treatment with MCSF in a concentration comprised between 35-55 nM, preferably 50 nM, more preferably 45 nM.

This step too may have a variable duration between 24 and 96 hours, preferably between 48 and 72 hours.

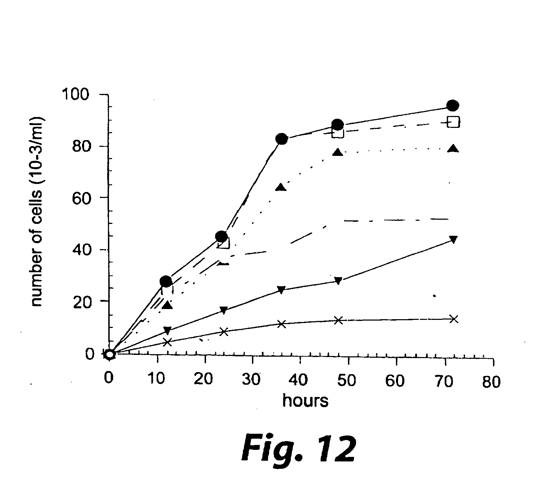

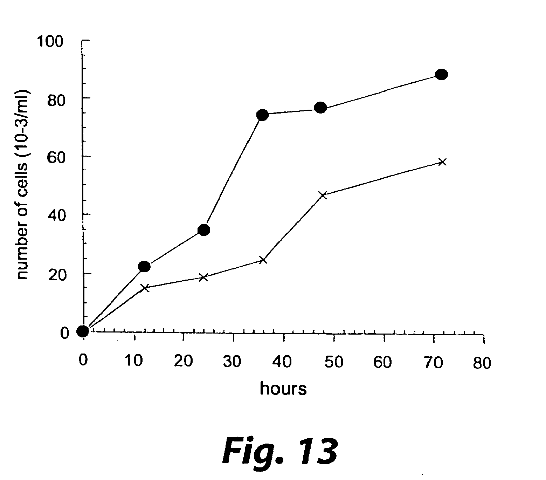

It has been observed that using MCSF in a concentration greater than 55 nM (i.e. 70 nM) already after 24 hours the cells do not keep the phenotype of pluripotent stem cells (see fig. 12). In particular, step a) of prior expansion in suspension with MCSF immediately after the blood has been taken allows to increase the percentage of stem cells with respect to the population of monocytes and macrophages (see fig. 13). The subsequent step allows to obtain pluripotent stem cells which are differentiated directly in vivo, without causing phenomena of rejection or infection.

The invention also concerns the use of the adult stem cells obtained according to the expansion method described above for the preparation of a medication for treating lesions. The lesions treatable are those of the group to which the following belong: cutaneous lesions, lesions to tendons, lesions to ligaments, bone lesions, lesions to the mucous membranes in mammals or fractures.

The present invention also concerns the use of adult stem cells obtained according to the method described above for the preparation of a medication for treating neurological or neurodegenerative pathologies chosen from the group that includes: Cushing's disease, head shaking, Wobbler's syndrome, breathing difficulties, paresis of the limbs; acute or chronic inflammatory pathologies chosen from laminitis, periostitis, gastritis, arthrosis, inflammations caused by viral, bacterial, parasite, mycotic agents; dilatation-torsion of the stomach in mammals.

The present invention also concerns a pharmaceutical composition comprising the adult stem cells obtained according to the expansion method described above as active principle, together with pharmacologically acceptable adjuvants and/or excipients.

According to a particularly preferred form of embodiment, the adult stem cells obtained according to the method defined above, used as active principle, are present in a concentration comprised between 90-250x10³ cells/ml in the composition, preferably 150x10³ cells/ml, together with the pharmacologically acceptable adjuvants and/or excipients, said composition being formulated for intravenous injection.

According to an alternative form of embodiment, the adult stem cells obtained according to the expansion method defined above, used as active principle, are present in a concentration comprised between 4-40x10⁶ cells/ml in the composition, together with the pharmacologically acceptable adjuvants and/or excipients, said composition being formulated for local application (also topical in the case of external wounds).

The pharmaceutical compositions mentioned above can also comprise an antibiotic as active principle in a concentration comprised between 5-15 nM, preferably 10 nM, according to a particularly preferred form of embodiment of the invention. Preferably said antibiotic is gentamicin or amicacin.

The invention also concerns the use of a pharmaceutical composition as defined above (i.e. suitable for local application, with or without antibiotic) as a medication for treating lesions chosen from the group consisting of cutaneous lesions, lesions to tendons, lesions to ligaments, bone lesions, lesions to the mucous membranes in mammals.

According to another feature, the invention concerns the use of the pharmaceutical composition as defined above (i.e. suitable for local application, with or without antibiotic) as a medication for treating fractures in mammals.

The present invention also concerns the use of a pharmaceutical composition as defined above (i.e. suitable for intravenous administration), as medication for treating neurological or neurodegenerative pathologies chosen from the group that includes: Cushing's disease, head shaking, Wobbler's syndrome, breathing difficulties, paresis of the limbs in mammals.

According to another form of embodiment the invention concerns the use of a pharmaceutical composition as defined above (i.e. suitable for intravenous administration), as medication for treating acute or chronic inflammatory pathologies chosen from laminitis, periostitis, gastritis, arthrosis, inflammations caused by viral, bacterial, parasite, mycotic agents in mammals.

The invention also concerns the use of a pharmaceutical composition as defined above (i.e. suitable for intravenous administration), as medication for treating the syndrome of dilatation-torsion of the stomach in mammals.

In a preferential form of embodiment of the present invention, the uses in the medical field have a preferable application in the veterinary field and the mammals treated are chosen from horses, dogs, cats or man.

The present invention will now be described as a non-restrictive example, in its preferred forms of embodiment, with particular reference to the attached drawings wherein:
Fig. 1 shows a lesion of 20 cm between the metatarsus and the first phalange with clostridia and destruction of the extensor tendon in a mare;
Fig. 2 shows the same mare of fig 1 three months after the local application of the stem cells according to the invention;
Fig. 3 shows the mare after six months of treatment;
Fig. 4 shows the ultrasound scan of a horse with a lesion of 80% of the surface flexor tendon;
Fig. 5 shows the ultrasound scan of the horse about three months and a half after the local treatment with stem cells according to the invention;
Fig. 6 shows the ultrasound scan of the horse about three months and a half after the local treatment with stem cells;
Fig. 7 shows the ultrasound scan of the horse after about four months of the local treatment; note the almost complete regeneration of the tendon and the lack of scar tissue;
Fig. 8 shows the ultrasound scan of a mare with a tendon lesion of lesser entity (less than 1 cm in diameter);
Fig. 9 shows the ultrasound scan of the same mare in fig. 7 after a month of the local treatment;
Fig. 10 shows a 17-year-old horse with general weakness, operated for colic, before treatment with the stem cells according to the invention;
Fig. 11 shows the performance of the 17-year-old horse one year after intravenous treatment with the stem cells according to the invention;
Fig. 12 shows the number of isolated cultured cells from peripheral blood treated with different quantities of MCSF in step c) of the present: 15 nM (broken line with crosses), 25 nM (broken line with triangles, apex down), 35 nM (broken line with triangles, apex up), 50 nM (broken line with black circles), 60 nM (broken line with white squares), 70 nM (broken line with white circles); the results are the average of cells counted from three samples of peripheral blood;
Fig. 13 shows the number of isolated cultured cells from peripheral blood pre-treated with MCSF (broken line with black dots), not treated with MCSF (broken line with crosses); the results are the average of cells counted from three samples of peripheral blood.

### EXAMPLE 1: Expansion and purification of adult stem cells from peripheral blood and their medical uses

The cells isolated from peripheral blood act "in vivo" as pluripotent stem cells (PSC) following the expansion according to the invention and are able to resolve, within the space of a few months, lesions or pathologies incurable or curable only slowly with classical methodologies and/or drugs.

### MATERIALS AND METHODS

### Sampling

Each sample of peripheral blood consisted of about 5-7 ml, it was taken from horses and dogs from the lower limbs and immediately put into test tubes containing, for example, heparin (150 U), and MCSF (10 nM).

However, the heparin can be replaced by another suitable anti-coagulant substance.

### Purification

The blood samples (5-7 ml) were diluted 1:5 in PBS (Phosphate Buffer Saline) containing NH₄Cl (200 mM), to cause the lyses of the red corpuscles, centrifuged at 10,000 g, washed twice with PBS and centrifuged again at 200 g. The nucleate cells obtained were incubated for 7-12 hours at 37°C, preferentially for 10-12 hours, and purified by means of fractioning on a Ficoll gradient, then isolated and washed three times with RPMI medium 1640 (Life Technologies, Grand Island, New York). Once purified, the cells that contained about 95% monocytes (as determined by means of cytofluorometric analysis by means of a FACScan-Becton Dickinson flow photometer), were incubated for another 24 hours in 50 ng/ml MCSF 45 nM and then expanded to obtain the number of cells necessary for the local treatments or centrifuged at 10,000 g and re-suspended in PBS at a concentration of about 90x10³ cells/ml for intravenous treatments.

### Cell cultures

The cells were incubated for 8-12 hours, preferentially for 10-12 hours, at 37°C (8% CO₂) on 15 cm plates at a concentration of about 2-3 x 10⁵ cells per plate; subsequently washed in culture medium to remove the non-adherent cells and then incubated another 48 hours with 10 ml of RPMI medium supplemented with 10% FBS, penicillin (100 units/ml), streptomycin (100 mg/ml), L-glutamine (2 mM) and MCSF (Macrophage Colony Stimulating Factor - 50 ng/ml). Some of these cells isolated with the stem cells show an elongated morphology, when incubated with MCFS, which can recall fibroblasts (Zhao Y. et al., 2003). All the products used, unless otherwise indicated, were obtained from Sigma-Aldrich. The cell suspension was obtained by pipetting after incubation for 5-8 minutes with 2% lidocaine (Sigma) in PBS as already described (Rabinovitch M. et al., 1976). Once detached, the cells were washed twice with PBS and centrifuged for 5' at 2000 g, added with gentamicin (10 nM) and diluted to a final concentration of 5-7x10⁶, preferentially 6x10⁶ cells/ml (except where a greater concentration is indicated).

In preliminary experiments a quota of cells was tested for the marker CD34, one of the main markers of hematopoietic stem cells by means of the techniques already described by Randall et al., 1998.

### Immunostaining

For cytophenotyping the cells were washed in PBS and then fixed on slides in formaldehyde 4% in PBS for 20 mins at 20°C.

To identify the intra-cellular proteins the cells were permeabilized with 0.5% Triton X-100 for 5 mins at 20°C and then incubated for 1 hour with the primary antibodies diluted in PBS containing 1% BSA (to block the aspecific antigenic sites). After three successive washes, the slides were then incubated for 45 mins with the secondary antibody conjugate with the most appropriate fluorochrome: FITC or tetramethylrhodamine B isothiocyanate (TRITC), or Cy5.

All the secondary antibodies were developed, using the donkey as host, by Jackson ImmunoResearch. The immunocytochemistries were carried out at the temperature of 4°C and in a humidity saturated atmosphere. After three washes, the slides were mounted using "gelvatol-PBS". The fluorescence images were then acquired by means of a fluorescence microscope using as internal standard an immunofluorescence directed against the glyceraldehyde 3-phosphate dehydrogenase (polyclonal sheep antibody produced by Cortex Biochem, San Leandro, CA). As negative controls and in order to calibrate the levels of the fluorescence background, slides incubated with aspecific antibodies were used, of the same isotype as the samples involved.

The images that are obtained show the cells seen through a phase contrast microscope, superimposed on the fluorescence image of the lipids colored with Nile Red and on the image of the nuclei colored with DAPI (4',6-diamidin-2-phenylindole). The reference bar measures 40 µm. The intensity of relative fluorescence was examined by means of quantitative ratio imaging microscopy between cells treated with MCSF and macrophages.

The method described above was used to identify all the markers investigated (CD 90, CD 34, CD90/CD34).

### RESULTS

### Local use

The cells were applied directly in the cases of external wounds. On the contrary, in the case of lesions to tendons, ligaments and fractures the cells were inoculated directly in the site of the lesion at a final concentration, except where otherwise indicated, of 5-10x10⁶ cells/ml according to the seriousness of the lesion; in cases where the cells could not be inserted precisely in the lesions the following method was used:
- for lesions to collateral ligaments the injection was made between the second and third phalange,
- for lesions to the naviculars the injection was made in the carpal tunnel,
- for lesions to the sacroiliac articulations and in cases of Wobbler, the injection was made between the fifth and the sixth cervical or between the sixth and the seventh.

### Cutaneous lesions

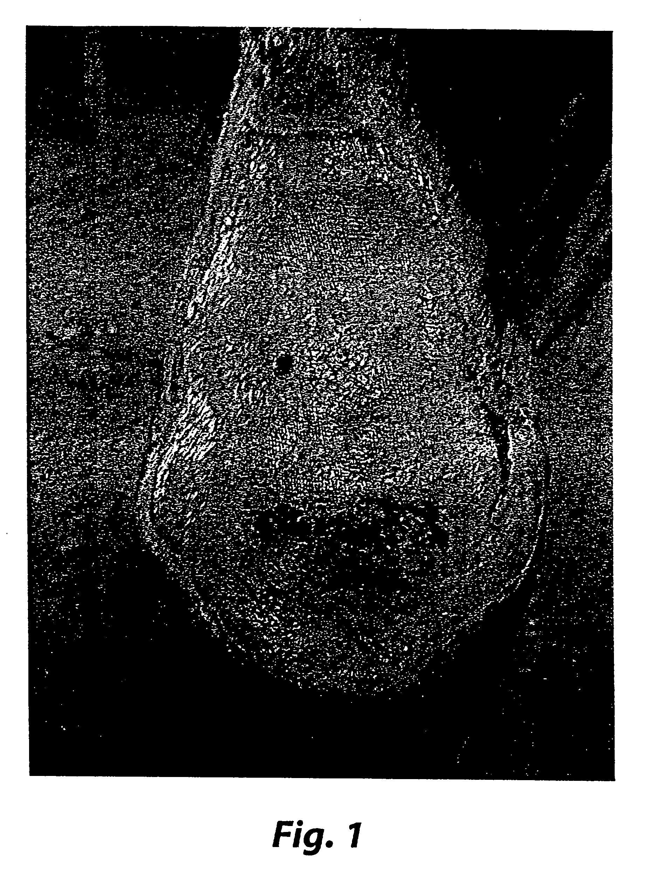

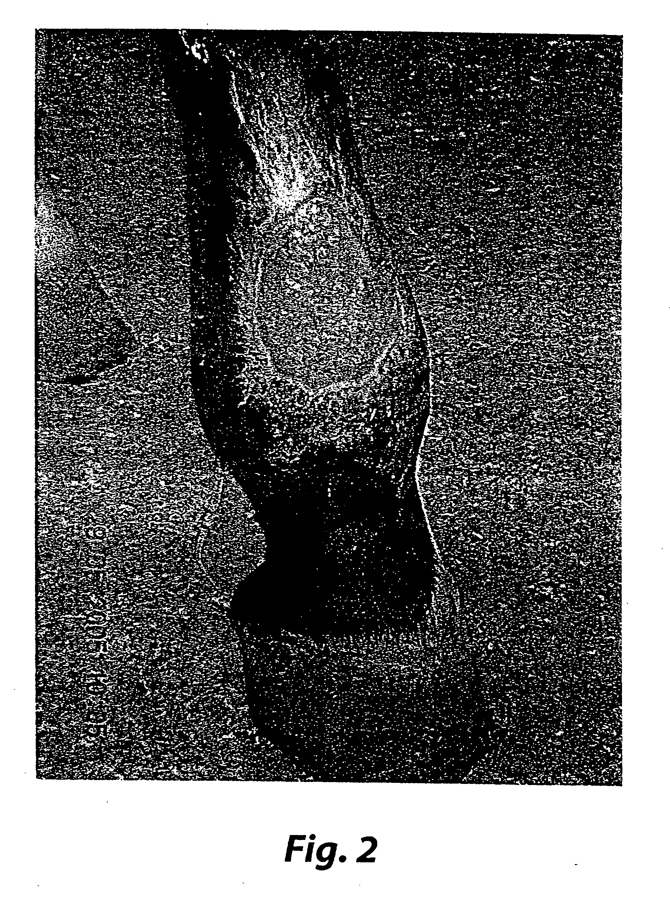

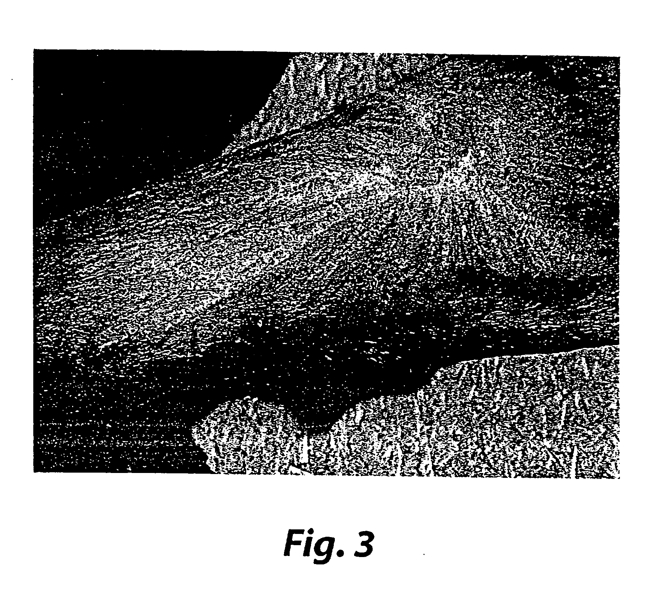

Case of a mare with a wound between metatarsus and first phalange, 20 cm in diameter with clostridia complications which had led to the destruction of the underlying tissues, including the extensor tendon (fig. 1). The first application was made one year after the accident and after two surgical operations to remove the cheloids, and was repeated three more times at a distance of 15 days, using from 10 to 400x10⁶ cells re-suspended in a physiological solution in the presence of gentamicin. After 100 days the wound was completely healed (fig. 2) and after 6 months the hair had re-appeared in 70% of the scar zone (fig. 3).

### Tendons

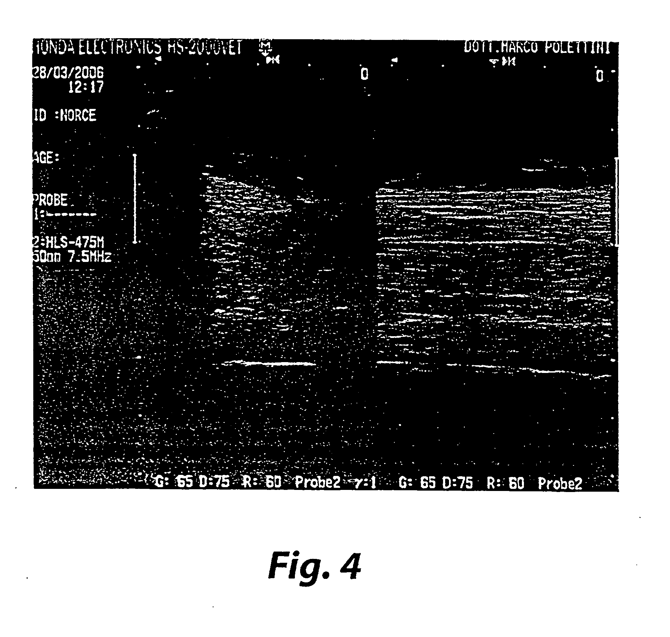

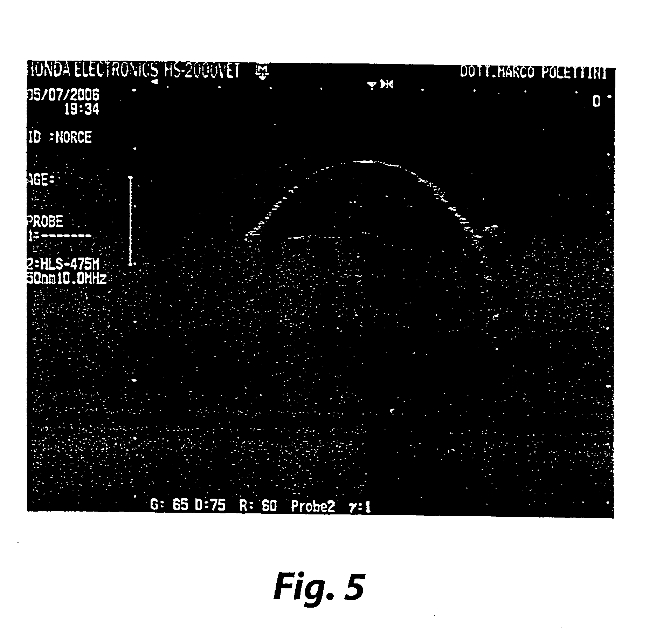

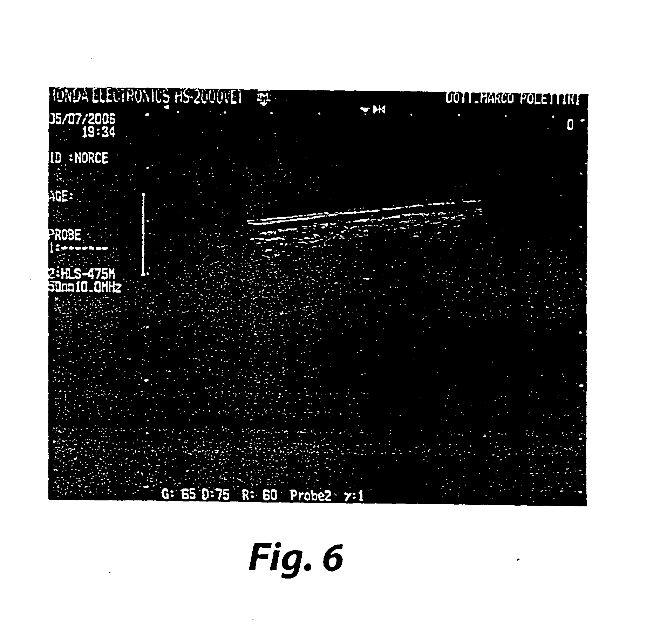

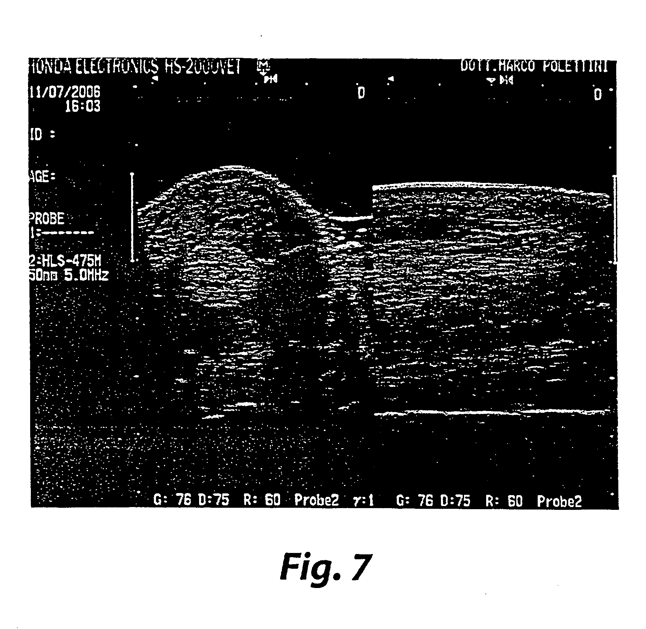

Three horses were treated with lesions of 80% of the superficial flexor tendon which already after three months of the treatment with about 300x10⁶ cells no longer showed hypoecogenic zones to an ultrasound exam and, on the contrary, showed that the thickness of the tendon (which had increased after the laceration and the inflammatory processes) was visibly reduced by 80%, as can be seen from the ultrasound scans shown in figs. 4-7.

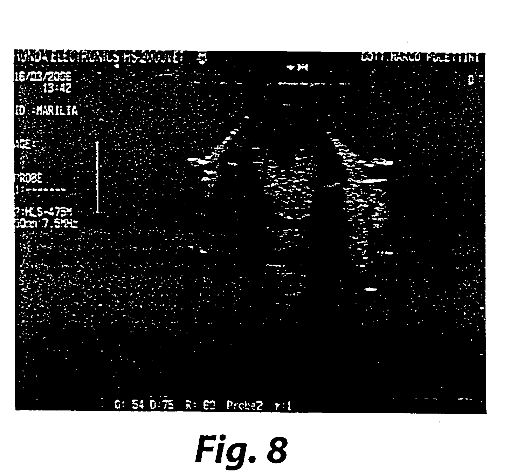

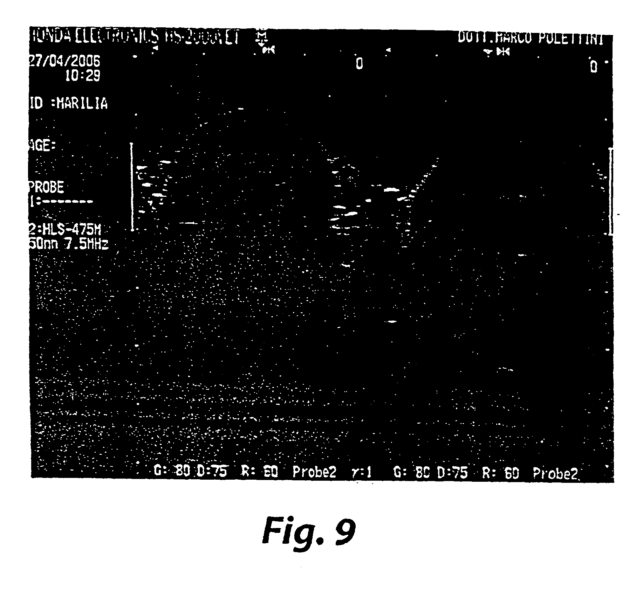

Other horses with smaller lesions to the tendon (1 cm in diameter) 1 month after the treatment no longer showed the lesion (figs. 8 and 9).

### Ligaments

Among the lesions treated we had an insertion of the suspender ligament under the hock with consequent lameness: less than three months after a local inoculation the horse starting working again, without lameness, and after one year there was no relapse.

Other horses treated, which had lesions between the branches and the central body of the suspender ligament, front and rear, were all resolved with *restitutio ad integrum.*

### Fractures

Among the fractures treated we had the case of a dog with a fractured femur which, following a surgical operation after 4 months was still not forming bone callus. After a local application of 1x10⁶ cells there was a complete recovery in 30 days.

### Mucous membranes

With regard to the healing of lesions to the mucous membranes, various chronic ulcers were treated. The most sensational case was that of a dressage horse with several ulcers in the mouth which had already had two plastic surgery operations. Following the treatment, the horse stopped bleeding already three days after local application of 4x10⁵ cells. After 15 days the lesions were completely healed.

In conclusion, the local application of isolated cells enriched with our method on tendons, ligaments, joints and fractures has shown more than 80% of the cases perfectly resolved within a few weeks or, at most, in four months. The remaining 20% of cases anyway showed considerable improvements. The methods commonly used today in veterinary science in these cases give positive results in not more than 60% of cases, after a treatment of about 6-15 months and improvements only in 5% of cases.

### Intravenous use

Cells were used intravenously (1 dose = 150x10³ cells) in the following pathologies:
Gushing's disease: this is a pathology due to hypertrophy of the intermediate hypophysis with reduced production of dopamine by the hypothalamus, very similar to Parkinson's disease in humans.

An affected pony was treated, which also had, apart from the classic symptoms of the disease, reduced immune defenses with hemolysis. After three treatments at a distance of 5 days with 150x10³ cells per treatment, improvements were noticed. After 4 cycles repeated at a distance of 40 days, the symptoms completely disappeared. Another four horses treated in the same way gave the same results.
Head shaking: neurological pathology of the central nervous system with secondary complications of the trigeminus which leads to continuous head shaking and problems of photophobia. The horse treated had had these symptoms for six months. The treatment entailed a cycle of 150x10³ cells at a distance of one week for 5 weeks. Already in the third week the symptoms had disappeared. Two other horses treated with the same protocol showed the same results.
Three cases of Wobbler: this is a congenital cervical neurological compression. The cases treated by administering three doses (intravenously and local) at a distance of a week, showed complete remission of the symptoms following treatment.

The intravenous use of expanded and purified stem cells using the method described showed how these cells are able "in vivo" to resolve pathologies affecting the neuronal tissue already giving the first results after a week of treatment.
Vascular reconstruction. In a horse affected by laminitis (destruction of the peripheral vascularization in the foot, with the result of an extremely painful lameness such as to prevent movement) the pain had almost disappeared 12-24 hours after a dose was administered, inoculated into the digital vessels. The same result was obtained for two other cases of horses affected by the same pathology.

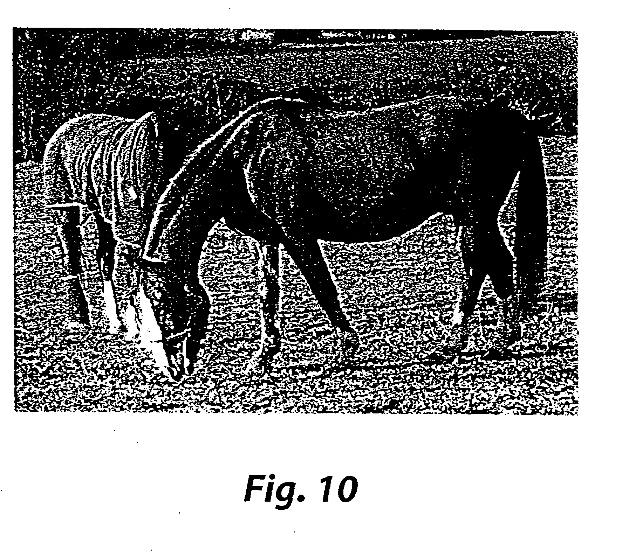

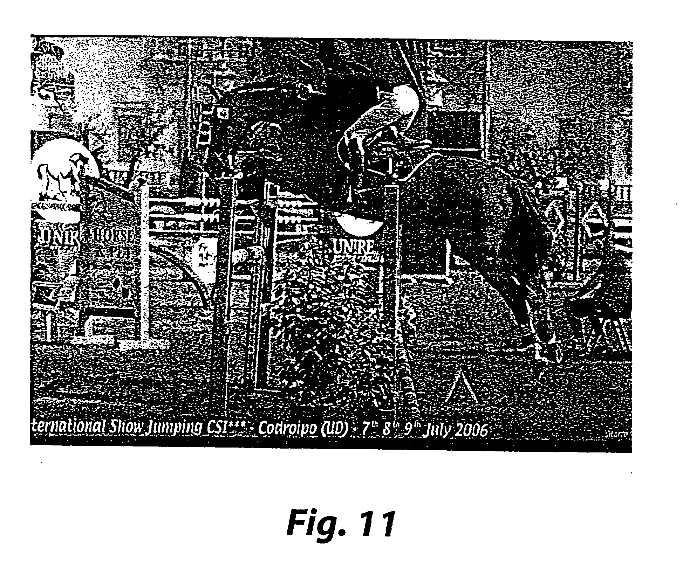

Other general cases where the cells were administered intravenously were:
- Case of a horse with a fracture of the navicular which restarted its competitive activity after the simultaneous administration of the stem cells in the peripheral vessels, the navicular bursa and the articular surface between the deep flexor tendon and the navicular bone;
- Case of a horse suffering from periostitis which, with the administration of two intravenous doses, improved its lameness;
- Case of a seventeen-year-old horse operated for colic and afterwards put to grass because no longer able to continue competitive activity due to a persistent state of general debilitation. After 4 cycles of 3 doses every 5 days, the horse started competitive activity again, participating in competitions and obtaining results it had never had before (figs. 10 and 11);
- Case of a twenty-year-old horse with suspected cerebral ischemia with consequent loss of coordination in three limbs which, after pharmacological treatment based on cortisone derivatives, still continued to keep an uncertain and staggering pace. After the administration of two doses at a distance of one week, the animal restarted its normal activity without showing any symptoms any longer;
- Case of a twenty-three-year-old mare, with a pregnancy at 21 years of age, which, after the period of lactation had finished, was treated with two doses of cells intravenously. After the treatment the mare restarted her competitive activity in full (at 20, a horse is considered too old for competitive activity);
- Case of a horse with a fractured pelvis which after two doses of cells restarted international competitive activity;
- Case of a fifteen-year-old horse with breathing difficulties and a very nervy character, which after treatment with two doses started competing again on an international level and without the slightest breathing difficulty;
- Case of two horses with extreme lameness, for more than two years consequent to the pulling of the collateral ligament between the first and second phalange. This pathology is considered irreversible in 75% of cases. After treatment with three doses for three months both animals treated returned to competing normally;
- Case of a thirteen-year-old horse operated for colic at 7 and castrated at 12. Following the second operation the animal had had non-emittent bacterial complications despite treatment with antibiotics and anti-inflammatories. Moreover, the animal suffered from chronic gastritis, proved by a gastroscopy. After the administration of 4 doses at a distance of one week, the horse was again given a gastroscopy which showed the complete regeneration of the gastric mucous membrane. The same result was obtained on 10 other gallop horses;
- Case of a horse with pansystolic murmur and numerous murmurs atypical to cardiac osculation, with blood test positive to an active form of Herpes virus 1 which caused neurological problems and loss of balance; in this case three intravenous doses were administered three times at a distance of 5 days, followed by a fourth dose administered at the level of the vertebral column. Already after two months the horse started to move again, almost completely regaining balance;
- Case of a nineteen-year-old horse almost completely retired from competitive activity due to a general physical deterioration, which after the administration of 200x10³ cells, two times at a distance of a week and with repeated treatment, after three months returned to being one of the best in his category (in jumping competitions, jumping heights of 1.35-1.40 m).

The same type of treatment was applied to very old dogs and gave the same results. In dogs the following pathologies were treated:
- two cases of dogs with torsion of the stomach: the first was a six-year-old great Dane subjected to a surgical operation but with a relapse after one week and therefore again subjected to another operation; after a first administration of 150x10³ cells the dog started eating again and after two doses at a distance of one week the dog returned to its normal activity; the second case was a ten-year-old female great Dane, suffering from arthrosis, diagnosed with diabetes, having had a hysterectomy-ovariectomy; four months after the operation, she had a torsion of the stomach with a subsequent surgical operation which, however, did not bring a great improvement. At this point, two doses were administered, at a distance of one week and four other cycles in the following four months. Today, eight months after the last cycle, the dog has not only a normal glycemia but also her ability to walk has improved by 80%;
- Case of a mongrel dog, 13 years old, male, with paresis of the rear limbs and incontinent; until that moment it had only been treated with cortisone, without appreciable results. Fifteen days after the cortisone was suspended, the cells were taken and the dog was subjected to a cycle of two doses at a distance of a week. Six days after the last treatment the dog had not only reacquired the use of its legs, but was urinating and defecating normally.

The results obtained with the method according to the invention make this procedure extremely versatile, thanks to the fact that, at the moment, no "in vivo" technique provides to expand pluripotent cells. Moreover, the lack of any form of rejection or infection following the administration in all the case histories reported above makes this technique suitable for auto-transplant procedures.

## Claims

1. Method for the expansion of adult stem cells from blood, particularly but not only peripheral blood, comprising the following steps:
a) a first step of expansion of the stem cells of blood, immediately after they have been taken from the patient, by means of the in-vitro treatment with MCSF in a concentration comprised between 8-15 nM and
b) a second step of purification of the expanded stem cell. c) expansion of the stem cells of blood purified in step b) by means of the in-vitro treatment with MCSF in a concentration comprised between about 35-55 nM for 24-72 hours.

2. Method as in claim 1, **characterized in that** said in-vitro step a) with MCSF has a duration comprised between 24 and 96 hours.

3. Method as in claim 1, **characterized in that** said in-vitro step a) with MCSF has a duration comprised between 48 and 72 hours.

4. Method as in claim 1, **characterized in that** the in-vitro treatment of step a) is started not more than 10 minutes after the sample has been taken, advantageously not more than 5 minutes.

5. Method as in any claim hereinbefore, wherein said concentration of MCSF in step a) is preferably 10 nM_{.}

6. Method as in claim 1, wherein said concentration of MCSF in step c) is preferably 50 nM, more preferably 45 nM.

7. Method as in any claim hereinbefore, wherein the in-vitro treatment with MCSF in step a) and/or step c) lasts 24-48 hours, preferably 45-48 hours.

8. Method as in any claim hereinbefore, wherein the purification step b) occurs by means of fractioning on a Ficoll gradient.

9. Pharmaceutical composition comprising the adult stem cells obtained according to the method as defined in any claim from 1 to 8 at a concentration comprised between 90-250x10³ cells/ml, preferably 100-120x10³ cells/ml, as active principle, together with pharmacologically acceptable adjuvants and/or excipients, said composition being formulated for intravenous injection, for use in the treatment of lesions chosen from the group consisting of cutaneous lesions, lesions to the tendons, to the ligaments, to the bones, to the mucous membranes in mammals, or fractures, of neurological or neurodegenerative pathologies chosen from the group consisting of Cushing's disease, head shaking, Wobbler's syndrome, breathing difficulties, paresis of the limbs in mammals, of acute or chronic inflammatory pathologies chosen from laminitis, periostitis, gastritis, arthrosis, inflammations caused by viral, bacterial, parasite, mycotic agents in mammals, of the syndrome of dilatation-torsion of the stomach in mammals.

10. Pharmaceutical composition comprising the adult stem cells obtained according to the method as defined in any claim from 1 to 8 at a concentration comprised between 4-40x10⁶ cells/ml, preferentially 7x10⁶ cells/ml, as active principle together with pharmacologically acceptable adjuvants and/or excipients, said composition being formulated for local application, for use in the treatment of lesions chosen from the group consisting of cutaneous lesions, lesions to the tendons, to the ligaments, to the bones, to the mucous membranes in mammals, or fractures, of neurological or neurodegenerative pathologies chosen from the group consisting of Cushing's disease, head shaking, Wobbler's syndrome, breathing difficulties, paresis of the limbs in mammals, of acute or chronic inflammatory pathologies chosen from laminitis, periostitis, gastritis, arthrosis, inflammations caused by viral, bacterial, parasite, mycotic agents in mammals, of the syndrome of dilatation-torsion of the stomach in mammals.

11. Composition for use as in claim 10, also comprising an antibiotic as an active principle in a concentration comprised between 5-15 nM, preferably 10 nM.

12. Composition for use as in claim 11, where the antibiotic is gentamicin.

13. Composition for use according to claim 9, wherein said composition comprises adult stem cells obtained at a concentration equal to 150x10³ cells/ml and is administered once a week intravenously.

14. Composition for use according to any claim from 9 to 13, wherein said mammals are chosen from among men, horses, cats or dogs.

## Patentansprüche

1. Verfahren zur Expansion adulter Stammzellen aus Blut, insbesondere aber nicht ausschließlich aus peripherem Blut, umfassend die folgenden Schritte:
a) einen ersten Schritt der Expansion der Stammzellen aus Blut durch in-vitro Behandlung mit MCSF in einer Konzentration zwischen 8-15 nM sofort nachdem sie aus einem Patienten entnommen wurden, und
b) einen zweiten Schritt der Reinigung der expandierten Stammzellen, und
c) Expansion der Stammzellen aus dem Blut, die in Schritt b) gereinigt wurden, durch in-vitro Behandlung mit MCSF in einer Konzentration zwischen ungefähr 35-55 nM für 24-72 Stunden.

2. Verfahren nach Anspruch 1, **dadurch gekennzeichnet, dass** der in-vitro Schritt a) mit MCSF eine Dauer zwischen 24 und 96 Stunden aufweist.

3. Verfahren nach Anspruch 1, **dadurch gekennzeichnet, dass** der in-vitro Schritt a) mit MCSF eine Dauer zwischen 48 und 72 Stunden aufweist.

4. Verfahren nach Anspruch 1, **dadurch gekennzeichnet, dass** die in-vitro Behandlung von Schritt a) nicht später als 10 Minuten, vorteilhafter Weise nicht mehr als 5, Minuten, nachdem die Probe entnommen wurde, gestartet wird.

5. Verfahren nach einem der vorhergehenden Ansprüche, **dadurch gekennzeichnet, dass** die Konzentration von MCSF in Schritt a) vorzugsweise 10 nM beträgt.

6. Verfahren nach Anspruch 1, **dadurch gekennzeichnet, dass** die Konzentration von MCSF in Schritt c) vorzugsweise 50 nM, besonders bevorzugt 45 nM beträgt.

7. Verfahren nach einem der vorhergehenden Ansprüche, **dadurch gekennzeichnet, dass** die in-vitro Behandlung mit MCSF in Schritt a) und/oder in Schritt c) 24-48 Stunden, vorzugsweise 45-48 Stunden andauert.

8. Verfahren nach einem der vorhergehenden Ansprüche, **dadurch gekennzeichnet, dass** die Reinigung in Schritt b) durch Fraktionierung mittels Ficoll-Gradienten erfolgt.

9. Pharmazeutische Zusammensetzung enthaltend adulte Stammzellen, die nach dem Verfahren nach einem der Ansprüche 1-8 gewonnen wurden, in einer Konzentration zwischen 90-250x10³ Zellen/ml, vorzugsweise 100-120x10³ zellen/ml, als aktiven Bestandteil, zusammen mit pharmazeutisch akzeptablen Adjuvantien und/oder Hilfsstoffen, wobei die Zusammensetzung so formuliert ist, dass sie zur intravenösen Injektion geeignet ist, zur Verwendung in der Behandlung von Läsionen ausgewählt aus der Gruppe bestehend aus Hautläsionen, Läsionen der Sehnen, der Bänder, der Knochen, der Schleimhäute in Säugetieren, oder Frakturen, oder neurologischen oder neurodegenerativen Pathologien, ausgewählt aus der Gruppe bestehend aus Morbus Cushing, Kopfwackeln, Wobbler Syndrom, Atmungsschwierigkeiten, Parese der Gliedmaßen bei Säugetieren, akuten oder chronisch entzündlichen Pathologien ausgewählt aus Laminitis, Knochenhautentzündung, Gastritis, Arthrose, Entzündungen verursacht durch virale, bakterielle, parasitäre, mykotische Erreger in Säugetieren, des Syndroms der Dilations-Torsion des Magens in Säugetieren.

10. Pharmazeutische Zusammensetzung enthaltend adulte Stammzellen, die nach dem Verfahren nach einem der Ansprüche 1-8 gewonnen wurden, in einer Konzentration zwischen 4-40x10⁶ Zellen/ml, vorzugsweise 7x10⁶ Zellen/ml, als aktiven Bestandteil, zusammen mit pharmazeutisch akzeptablen Adjuvantien und/oder Hilfsstoffen, wobei die Zusammensetzung so formuliert ist, dass sie zur lokalen Anwendung geeignet ist, zur Verwendung in der Behandlung von Läsionen ausgewählt aus der Gruppe bestehend aus Hautläsionen, Läsionen der Sehnen, der Bänder, der Knochen, der Schleimhäute in Säugetieren, oder Frakturen, oder neurologischen oder neurodegenerativen Pathologien, ausgewählt aus der Gruppe bestehend aus Morbus Cushing, Kopfwackeln, Wobbler Syndrom, Atmungsschwierigkeiten, Parese der Gliedmaßen bei Säugetieren, akuten oder chronisch entzündlichen Pathologien ausgewählt aus Laminitis, Knochenhautentzündung, Gastritis, Arthrose, Entzündungen verursacht durch virale, bakterielle, parasitäre, mykotische Erreger in Säugetieren, des Syndroms der Dilations-Torsion des Magens in Säugetieren..

11. Zusammensetzung zur Verwendung nach Anspruch 10, ferner umfassend ein Antibiotikum als aktiven Bestandteil in einer Konzentration zwischen 5-15 nM, vorzugsweise 10 nM.

12. Zusammensetzung zur Verwendung nach Anspruch 11, wobei das Antibiotikum Gentamicin ist.

13. Zusammensetzung zur Verwendung nach Anspruch 9, wobei die Zusammensetzung adulte Stammzellen umfasst, gewonnen in einer Konzentration, die 150x10³ Zellen/ml entspricht, und die einmal in der Woche intravenös verabreicht wird.

14. Zusammensetzung zur Verwendung nach einem der Ansprüche 9 bis 13, wobei die Säugetiere ausgewählt sind aus Menschen, Pferden, Katzen und Hunden.

## Revendications

1. Procédé d'expansion de cellules souches adultes de sang, en particulier mais non uniquement de sang périphérique, comprenant les étapes suivantes :
a) une première étape d'expansion de cellules souches de sang, immédiatement après leur prélèvement sur le patient, au moyen du traitement in vitro avec du MCSF à une concentration comprise entre 8 et 15 nM,
b) une deuxième étape de purification des cellules souches expansées et
c) l'expansion des cellules souches de sang purifiées à l'étape b) au moins du traitement in vitro avec du MCSF à une concentration comprise entre environ 35 et 55 nM pendant 24 à 72 heures.

2. Procédé selon la revendication 1, **caractérisé en ce que** ladite étape in vitro a) avec du MCSF a une durée comprise entre 24 et 96 heures.

3. Procédé selon la revendication 1, **caractérisé en ce que** ladite étape in vitro a) avec du MCSF a une durée comprise entre 48 et 72 heures.

4. Procédé selon la revendication 1, **caractérisé en ce que** le traitement in vitro de l'étape a) est commencé au plus 10 minutes après que l'échantillon a été prélevé, avantageusement au plus 5 minutes après.

5. Procédé selon l'une quelconque des revendications précédentes, dans lequel ladite concentration de MCSF dans l'étape a) est de préférence de 10 nM.

6. Procédé selon la revendication 1, dans lequel ladite concentration de MCSF dans l'étape c) est de préférence de 50 nM, plus préférablement de 45 nM.

7. Procédé selon l'une quelconque des revendications précédentes, dans lequel le traitement in vitro avec du MCSF à l'étape a) et/ou à l'étape c) dure de 24 à 48 heures, de préférence de 45 à 48 heures.

8. Procédé selon l'une quelconque des revendications précédentes, dans lequel l'étape de purification b) se déroule par fractionnement sur un gradient de Ficoll.

9. Composition pharmaceutique comprenant les cellules souches adultes obtenues selon le procédé tel que défini dans l'une quelconque des revendications 1 à 8 à une concentration comprise entre 90 et 250.10³ cellules/ml, de préférence entre 100 et 120.10³ cellules/ml, en tant que principe actif, avec des adjuvants et/ou des excipients pharmacologiquement acceptables, ladite composition étant formulée pour une injection intraveineuse, pour une utilisation dans le traitement de lésions choisies dans le groupe constitué par les lésions cutanées, les lésions des tendons, des ligaments, des os, des membranes muqueuses chez les mammifères et les fractures, les pathologies neurologiques ou neuro-dégénératives choisies dans le groupe constitué par la maladie de Cushing, le tic à l'encensé, le syndrome de Wobbler, les difficultés respiratoires, la parésie des membres chez les mammifères, les pathologies inflammatoires aigües ou chroniques choisies parmi la fourbure, la périostite, la gastrite, l'arthrose, les inflammations causées par des agents viraux, bactériens, parasites ou mycotiques chez les mammifères et le syndrome de la dilatation-torsion de l'estomac chez les mammifères.

10. Composition pharmaceutique comprenant les cellules souches adultes obtenues par le procédé tel que défini dans l'une quelconque des revendications 1 à 8 à une concentration comprise entre 4 et 40. 10⁶ cellules/ml, de préférence de 7.10⁶ cellules/ml, en tant que principe actif avec des adjuvants et/ou des excipients pharmacologiquement acceptables, ladite composition étant formulée pour une application locale, pour une utilisation dans le traitement de lésions choisies dans le groupe constitué par les lésions cutanées, les lésions des tendons, des ligaments, des os, des membranes muqueuses chez les mammifères et les fractures, les pathologies neurologiques ou neuro-dégénératives choisies dans le groupe constitué par la maladie de Cushing, le tic à l'encensé, le syndrome de Wobbler, les difficultés respiratoires, la parésie des membres chez les mammifères, les pathologies inflammatoires aigües ou chroniques choisies parmi la fourbure, la périostite, la gastrite, l'arthrose, les inflammations causées par des agents viraux, bactériens, parasites ou mycotiques chez les mammifères et le syndrome de la dilatation-torsion de l'estomac chez les mammifères.

11. Composition pour une utilisation selon la revendication 10, comprenant en outre un antibiotique en tant qu'ingrédient actif à une concentration comprise entre 5 et 15 nM, de préférence de 10 nM.

12. Composition pour une utilisation selon la revendication 11, dans laquelle l'antibiotique est la gentamicine.

13. Composition pour une utilisation selon la revendication 9, dans laquelle ladite composition comprend des cellules souches adultes obtenues à une concentration égale à 150.10³ cellules/ml et est administrée une fois par semaine de manière intraveineuse.

14. Composition pour une utilisation selon l'une quelconque des revendications 9 à 13, dans laquelle lesdits mammifères sont choisis parmi les hommes, les chevaux, les chats et les chiens.
